# EUROPEAN PATENT SPECIFICATION

(11) **EP 0 914 163 B1**
(45) Date of publication and mention of the grant of the patent: **24.02.2016**
(21) Application number: 98912887.1
(22) Date of filing: 26.02.1998
(51) Int. Cl.: A61K 35/38, A61K 39/00, A61P 1/00, A61P 37/00

(54) **Processes implementing selective immune down regulation (SIDR)**
Verfahren zur selektiven Immunherabregulation (SIDR)
Procédé de sous-régulation immunitaire sélective (SIDR)

(30) Priority: 28.02.1997 US 808629
(43) Date of publication of application: 12.05.1999
(60) Divisional application: 10184206.0
(73) Proprietor: ENZO THERAPEUTICS, INC., New York, NY 10022 (US)
(72) Inventor: ROY-CHOWDHURY, Jayanta, New Rochelle, NY 10804 (US); ILAN, Yaron, Jerusalem (IL); RABBANI, Elazar, New York, NY 10003 (US); ENGELHARDT, Dean, L., New York, NY 10025 (US)
(74) Representative: Vossius & Partner Patentanwälte Rechtsanwälte mbB
(86) International application number: PCT/US1998/003606
(87) International publication number: WO 1998/037917

(56) References cited:
- WO-A-92/06704
- WO-A-95/27499
- WO-A-95/27500
- US-A- 4 322 405
- US-A- 5 399 347
- ILAN Y ET AL: "Oral tolerization to adenoviral antigens permits long-term gene expression using recombinant adenoviral vectors." HEPATOLOGY, vol. 24, no. 4 part 2, October 1996 (1996-10), page 247A XP000990487 47th Annual Meeting and Postgraduate Courses of the American Association for the Study of Liver Diseases; Chicago, Illinois, USA; 8-12 November 1996
- ILAN Y ET AL: "Oral tolerization to adenoviral antigens permits long-term gene expression using recombinant adenoviral vectors." JOURNAL OF CLINICAL INVESTIGATION, vol. 99, no. 5, 1 March 1997 (1997-03-01), pages 1098-1106, XP000990152
- CARR R I ET AL: "Feeding donor spleen cells both before and after transplantation significantly prolongs renal allograft survival." FASEB JOURNAL, vol. 10, no. 6, 1996, page A1023 XP002163638 Joint Meeting of the American Society for Biochemistry and Molecular Biology, the American Society for Investigative Pathology and the American Association of Immunologists; New Orleans, Louisiana, USA; 2-6 June 1996
- MOLLER et al., "Cellular Mechanisms Underlying Differential Rejection of Sequential Heart and Lung Allografts in Rats", TRANSPLANTATION, March 1993, Vol. 55, Number 3, pages 650-655, XP002912007
- THOMAS et al., "Beneficial Effect of Cyclosporine in Posttransplantation Induction of Unresponsiveness of Renal Allografts in Rhesus Monkeys", TRANSPLANTATION PROCEEDINGS, February 1988, Vol. XX, No. 1, Suppl. 1, pages 134-136, XP002912008
- SMITH et al., "New Approaches to Transplantation Tolerance", TRANSPLANTATION PROCEEDINGS, August 1994, Vol. 23, No. 4, pages 2157-2161, XP002912009
- ROSENGARD et al., "Induction of Specific Tolerance to Class I-Disparate Renal Allografts in Miniature Swine with Cyclosporine", TRANSPLANTATION, September 1992, Vol. 54, No. 3, pages 490-497, XP002912010
- SYKES et al., "Bone Marrow Transplantation as a Means of Inducing Tolerance", SEMINARS IN IMMUNOLOGY, 1990, Vol. 2, pages 401-417, XP002912011
- SHARABI et al., "Specific Tolerance Induction Across a Xenogeneic Barrier: Production of Mixed Rat/Mouse Lymphohematopoietic Chimeras Using a Nonlethal Preparative Regimen", THE JOURNAL OF EXPERIMENTAL MEDICINE, July 1990, Vol. 172, pages 195-202, XP002912012
- PEARSON et al., "Induction of Transplantation Tolerance in Adults using Donor Antigen and Anti-CD4 Monoclonal Antibody", TRANSPLANTATION, September 1992, Vol. 54, No. 3, pages 475-483, XP002912013
- MADSEN et al., "Immunological Unresponsiveness Induced by Recipient Cells Transfected with Donor MHC Genes", NATURE, March 1988, Vol. 332, pages 161-164, XP002912014
- GUZZETTA et al., "Induction of Kidney Transplantation Tolerance Across Major Histocompatibility Complex Barriers by Bone Marrow Transplantation in Miniature Swine", TRANSPLANTATION, April 1991, Vol. 51, pages 862-866, XP002912015
- GOTSMAN I ET AL: "Amelioration of immune-mediated experimental colitis: tolerance induction in the presence of preexisting immunity and surrogate antigen bystander effect.", THE JOURNAL OF PHARMACOLOGY AND EXPERIMENTAL THERAPEUTICS, vol. 297, no. 3, June 2001 (2001-06), pages 926-932, ISSN: 0022-3565
- DASGUPTA A ET AL: "Oral administration of unmodified colonic but not small intestinal antigens protects rats from hapten-induced colitis.", CLINICAL AND EXPERIMENTAL IMMUNOLOGY, vol. 125, no. 1, July 2001 (2001-07), pages 41-47, ISSN: 0009-9104
- HANAUER S B: "Inflammatory bowel disease.", THE NEW ENGLAND JOURNAL OF MEDICINE, vol. 334, no. 13, 28 March 1996 (1996-03-28), pages 841-848, ISSN: 0028-4793

## Description

### FIELD OF THE INVENTION

This invention relates to the field of immunology and to novel processes for the modulation of immune responses including particularly the down regulation of the immune response system using procedures or combinations of procedures for producing and applying a new and unexpected immune modulation termed selective immune down regulation.

### BACKGROUND OF THE INVENTION

While immunological responses are essential in animals and man, certain undesirable consequences of these responses may occur and often do. A partial list of examples of such undesirable responses include autoimmune disease, serum sickness, rejection of cell, tissue and organ transplants, rejection of desirable nonnative components, immune complex-based destruction of certain tissues, tissue and cell destruction based on the induction of apoptosis or cell death, certain actions of antiidiotypic antibodies and certain anaphylactic responses.

One aspect of this problem revolves around the unwanted immune response to the production or presentation of non-native compounds, proteins or other antigenic materials (not found in the subject in which they have been placed) or proteins or other antigenic materials which are native constituents of the subject, but for some reason have been rendered immunogenic. In the latter instance, these specific proteins or other antigenic materials could have been rendered immunogenic for a number of reasons. They may have been modified to express a new antigenic determinant. An aberrant site of expression may have developed or even the amount of these proteins may have been altered rendering them immunogenic.

Unwanted host immune response can occur in the course of gene therapy. The immune response can be directed against antigens present in the vector and/or the products of the transferred genes. In general, the undesired production or presentation of antigens can result from the use of any viral or non-viral gene delivery system. Such an immune response can shorten the duration of expression of transgenes and can substantially reduce or inhibit a repeat of the transduction to reinstate these genes, thus posing a major hurdle to long-term gene therapy.

While dependence and reliance on immune response is both necessary and required, strategies to prevent or to overcome the undesirable consequences of certain immunological responses, are limited, and often ineffective. In general, immunological suppressive methods and procedures lead to overall suppression of the immune system. Maintaining a prolonged state of immunological suppression by overall suppression of the immune system is not desirable if it can be avoided.

In contrast to general immunosuppression, tolerance to specific antigens (such as adenovirus particles) can be induced if the antigen is injected into a neonate or into a fetus (Takahashi et al., J Biol Chem:271:26536-26534 (1996)); (Hagstrom, et al., Proc Natl Acad Sci 93:3056-61 (1996)). However, this procedure has a major limitation. It is effective only in the fetus or during the first few days after birth (not an adult).

Another tolerization protocol involves the direct injection of a soluble antigen into the functional thymus. (Ilan, et al., J Clin Invest, 98:2640-2647 (1996). This modality of tolerization is not applicable to adult subjects since such subjects lack an active thymus (not an adult).

Certain infections could lead to an autoimmune response in which both infected and/or uninfected cells are subjected to an undesirable immune response. Examples of such responses are hepatitis B infection, HIV infection and rheumatic fever. Because immune response complications are intermingled with the element of infection, there is currently no effective cure or management strategy for these diseases.

Furthermore, the use of many non-native compounds (adenovirus, for example) that are immunogenic in a subject, is also limited or inhibited due to the immunological response of the subject to these compounds and reagents.

Immunological modulation is an artificially induced variation in a subject's immune system in response to the introduction of reagents, procedures and processes. Such modulation could be based on an immune response that is humoral or cellular or both which in turn occurs in response to a non-native compound. Immunological modulation could be used to suppress an immunological response broadly or narrowly.

### SUMMARY OF THE INVENTION

A novel use for producing selective immune down regulation (SIDR) and immune suppression in subjects is provided by this invention.

This invention provides colon-extracted proteins from allogeneic and autologous sources for use in the treatment of Crohn's disease.

More particular details of the invention are described more fully below in the detailed description and example sections of this application that follow.

### BRIEF DESCRIPTlON OF THE FIGURES

**FIGURE 1** demonstrates β-galactosidase expression in liver specimens from orally tolerized rats (group B) and the control rats (Group C2) after the second injection of the recombinant virus.
**FIGURE 2** shows PCR gel results from the detection of the presence of human BUGT₁ DNA in rat livers after the second injection of the recombinant virus.
**FIGURE 3** is a Western blot analysis of expression of human BUGT₁ after the second injection of the recombinant virus.
**FIGURE 4** shows the results of the effect of tolerization upon bilirubin levels after the second injection of the recombinant virus.
**FIGURE 5** depicts the anti-adenovirus antibody levels in group A tolerized (solid bars) and group C control (open bars) rats after the first and second injection of the recombinant virus.
**FIGURE 6** are micrographs of liver biopsies taken taken 24-72 hours after the second injection showing minimal lymphocytic infiltration in tolerized rats (A) and severe inflammation in the control rats (B).
**FIGURE 7** are PCR gel results from the detection of the presence of human BUGT₁ DNA in rat livers after the second injection of the recombinant virus.
**FIGURE 8** is a Western blot analysis of expression of human BUGT₁ after the second injection of the recombinant virus.
**FIGURE 9** is a graph showing the effect of tolerization upon bilirubin levels after the second injection of the recombinant virus.
**FIGURE 10** is a graph showing serum bilirubin levels after adoptive transfer.
**FIGURE 11** is a color micrograph for β-galactosidase expression in liver specimens from rabbits after first injection.
**FIGURE 12** is also a color micrograph for β-galactosidase expression in liver specimens from rabbits three weeks after first injection.
**FIGURE 13** is also a color micrograph for β-galactosidase expression in liver specimens from orally tolerized rabbits after second injection.
**FIGURE 14** is also a color micrograph of β-galactosidase expression in liver specimens from non-tolerized control rabbits after second injection.

### DETAILED DESCRIPTION OF THE INVENTION

The present invention provides, as defined in the claim, a new immune modulating process in which the immune response system of a subject can be specifically down regulated. This novel approach to immune modulation in which undesirable or deleterious immune reactions are specifically suppressed in a subject has been termed selective immune down regulation or SIDR.

Another aspect is to improve such a process by combining at least one oral tolerization procedure with any other immune modulation procedures which could even include two or more such immune modulating procedures or treatments. Such combinations could take the form of two independent and separate selective immune down regulation procedures both directed to a given specific antigens or antigens. For example, two such selective immune down regulation procedures could comprise oral tolerization and selective immune suppression. Such a procedure could further comprise the use of other immune modulating procedures such as immunosuppressive drugs, appropriate cytokines, adjuvants, conjugates, or combinations thereof.

As used herein, "selective immune down-regulation" (SIDR) is an immunological state in a subject or biological system in which the subject maintains tolerance (prevention or suppression of a specific immune response) to a particular antigen or set of antigens (or other immunological determinant(s)) while at the same time maintaining immunological competence against other antigens, or other classes of antigens or immunological determinants. Furthermore, in such a state, SIDR is capable of being maintained in the subject after immunological processes or modulation that has led to the SIDR state has ceased or terminated.

As used herein, the term "dominant" as employed with immune down regulation or DIDR refers to a particular form of SIDR. If the SIDR state can be transferred and manifested as a dominant state in the new subject, then such a state is defined as a dominant immune down-regulation (DIDR)-state.

As used herein, the term "general immune suppression or suppressives" (GIS) refers to immune modulating reagents or procedures which could lead to the prevention of an immune response that is not specific to any particular antigen or set of antigens but rather is indiscriminate, non-specific and general. Such an immune suppression can be maintained in general if the reagents or procedures are themselves maintained. Such reagents or procedures can be administered transiently, repeatedly, or over prolonged duration.

Introduction of the SIDR producing reagent or combination of reagents can be carried out using conventional methodology and procedures that are well known to those skilled in this art. For example, the reagent or combinations can be introduced continuously into the subject, or introduced in a series of separate administrations. The separate administrations may be marked by fixed time intervals or variable time intervals, as the case may be. For a further description of administration and protocols for introducing the reagents or combination of reagents, reference is made to Oral Tolerance: Mechanisms and Applications, H. L. Weiner and L.F. Mayer, eds. (1996) The New York Academy of Sciences, New York, N.Y.'

One useful application described is the use of SIDR in combination with other conventional immune modulating treatments. The general immunological suppression procedure can take the form of immunosuppresive drugs, such as cyclosporin or other such drugs, or antibodies to immune cells such as anti-CD4, anti-CD8, OTK, etc. or cytokines or sub-ablative doses of radiation. Immunologic modulation leading to SIDR includes development of specific tolerance by use of general immune suppressors such as CD4 or CD8 antibodies or a combination with other anti-lymphocyte antibodies. While exposing the subject to continuous presence of specific antigenic immunosuppressive compounds and drugs that are well described in the literature. See for example, Benjamini's Immunology: A Short Course, 2nd Edition, Wiley-Liszt, Inc. (1991), Chapter 19 "Transplantation Immunology," pp. 347-367; and Stites' Basic and Clinical Immunology, 7th Edition, Appleton & Lange, Norwalk, CT (1991), Chapter 61 "Immunosuppressive Therapy," pp. 766-779. For more recent textbooks on this subject, please refer to Rich's Clinical Immunology: Principles and Practice, Mosby, St. Louis, MO, and Samter's Immunologic Diseases, 5th Edition, Little, Brown and Company, Boston.

T immunoregulatory cells (Ts cells) are induced either by certain antigen determinants or by the presence of specific allotypic or idiotypic determinants. Once these cells are induced, they act as memory cells capable of being reactivated throughout the lifetime of the host organism or the adaptive organism (Ilan, et al., Hepatology, 24:304,A 1996).

It should not in any way be overlooked that SIDR can be effected or obtained by means of oral tolerization. Even more significant is the principle or observation that SIDR or DIDR can also be effected through a selective immune suppressive. Such a selective immune suppressive (SIS) can comprise any of the following immune suppressors: an antibody to a T cell, an immune suppressive drug, and a cytokine or any combination thereof. The antibody to the T-cell can be representatively selected from the following: anti-CD4, anti-CD8 and OTK, or combinations thereof. Those skilled in the art will certainly appreciate that the foregoing short list of selective immune suppressives and T-cell antibodies are exemplary and by no means exhaustive.

In another aspect, a use for producing selective immune down regulation in a subject to a native antigen or group of native antigens (e.g., autoimmune antigens). In this case, the use comprises subjecting said subject to at least two separate immune modulating treatments at least one of which comprises oral tolerization. As part of this use, the native antigen or group of native antigens are derived from the subject's cell or tissue, or fragments thereof, or from the subject's cell or tissue or fragments complexed with antibodies, or from partial digests of any of the foregoing. Among representative antigens or group of antigens are those selected from collagen, islet cell, liver cell, kidney cell, heart cell, pancreatic cells, spleen cell, and nucleic acid, or combinations of the foregoing. The antigens or group of antigens can also comprise a cell or tissue (for example, bone marrow) organ or components or fragments thereof. Such things can be derived or taken from the donor's skin. The second treatment in the just described use can be selected to form SIDR, GIS or anti-apoptosis. SIDR can even be used for both or all of the separate immune modulating treatments.

In the last described use, one or more cytokines may be administered to the subject, or treatments may be administered as described earlier, for example, the separate immune modulating treatments can be given repeatedly in a single dosage or single dosage period or they can be given in separate dosage periods. In addition, the use in treatments can be given separately or concurrently with each other. Or they can be given with partial overlap in the dosage period. It should be noted that in implementing this use and applying it to a particular case, the subject (including mammals and humans) can be sensitive or naive to the antigen or group of antigens in question.

The use in the treatment provided herein is particularly useful for treating indications associated with autoimmune disease. Treatments that utilize tolerization for the treatment of autoimmune disorders are often severely limited by the requirement that tolerizing agents should be obtained from the afflicted individual. A further frequent limitation of current procedures, such as for the treatment of autoimmune diseases of the bowel, is the requirement for preventive use, by prior establishment of tolerization (Sharmanov, A. T. et al. (Gastroenterology 106(1994) A772; Neurath, M.F. et al. J. Exp. Med. 183(1996) 2605-2616). While such preventive measures can reduce the severity of autoimmune responses, they are not therapeutic, and thus are of little benefit to afflicted individuals. As described herein, selective immune down-regulation (SIDN) can be utilized to overcome these limitations by providing both for tolerizing agents that can be conveniently derived from allogeneic end xenogeneic sources as well as autologous sources and for their therapeutic use in the treatment of afflicted individuals.

As also disclosed herein, SIDR can be provided to individuals who are free of disease symptoms in order to provide a means of preventing IBD, or such treatments can be used therapeutically for the treatment at the onset of disease symptoms or concurrent with the presence of disease symptoms. SIDR can also be used in combination with other forms of therapy, such as generalized immune suppression as described in this application.

As, also described herein, tolerizing agents that can be utilized for such SIDR can be obtained from a number of sources including, for example, afflicted individuals as well as from other normal or afflicted individuals, from cadaver tissue or from animal tissues or from other sources that provide compounds that possess useful antigenic and/or immunological properties. Useful compounds for this purpose include diseased or normal tissues that can be derived from the afflicted individual or from other individuals, from cadaver tissue or from animal tissues. Such tolerizing agents can be used as whole tissues or extracts thereof including proteins, glycoproteins, peptides or glycopeptides or any combination thereof in crude or pure forms. Tolerizing agents can be modified by the addition of haptens and/or by proteolytic treatments. Such proteins can also be prepared synthetically or by the methods of recombinant DNA. Such compounds can be administered by oral routes, by injection or infusion into the stomach, or by other methods as described in this disclosure.

The compositions and uses of the present invention can provide for SIDR for the treatment of inflammatory bowel diseases such as ulcerative colitis and Crohn's disease, the principal inflammatory diseases of the gastrointestinal tract. While studies in mice show that symptoms of inflammatory bowel disease can be reduced by oral administration of an antigenic compound (trinitrobenzenesulfonic acid, TNBS) that is responsible for induction of the autoimmune process that leads to IBD, such treatment was administered to healthy, symptom-free animals prior to the induction of IBD (Sharmanov, A. T. *et al.,* 1994; Neurath, M.F. *et al.,* 1996). As disclosed herein SIDR can provide for marked decreases in the severity of such symptoms when treatment is administered in a therapeutic mode at or after the onset of disease symptoms. This can be achieved by the administration of antigenic compounds involved in the autoimmune response responsible for IBD or by compounds that possess similar antigenic and/or immunological properties. Such compounds can be administered by oral routes, by injection or infusion into the stomach, the ileum or by other routes all as provided or disclosed herein. Useful tolerizing agents for this purpose include diseased or normal tissues such as colonic tissues wherein such tissues can be derived from the afflicted individual or from other normal or afflicted individuals.

The use of SIDR to treat IBD can be demonstrated in rat studies by the feeding of proteins extracted from colons of colitis-afflicted rats to rats in whom colitis had been induced by intracolonic installation of trinitrobenzenesulfonic acid. Marked reduction of symptoms was observed when such SIDR was administered to rats at the same time that induction of colitis was administered. Compared to control rats that received no oral tolatization but had been treated to induce colitis, tolerized rats demonstrated marked decreases in the severity of the colitis symptoms of diarrhea, colonic ulceration, intestinal and peritoneal lesions, wall thickness and edema.
autologous source, but can be more conveniently obtained from allogeneic or xenogenic sources. Such tolerizing agents can thus include fat cells and pancreatic cells including islet cells, extracts of such cells and tissues including protein preparations. Such proteins or fractions thereof can include sialoglycolipid, glutamate decarboxylase, insulin receptor, 38 kd antigen, bovine serum albumin, glucose transporter, carboxypeptidase H, insulin receptors end others.

Compositions and methods for SIDR as provided herein may reduce or eliminate the symptoms of graft versus host disease (GVHD). For such uses in the treatment tolerization of the donor against the major histocompatibility and/or other antigens of the recipient can render the donor's immune cells tolerant of the cellular material of the recipient. An example of such induction of immune tolerance as a means of providing for reduction of the symptoms of GVHD is presented herein where such immune tolerance is established in mice by feeding donor mice with recipient lymphocytes that overexpress MHC class **II** antigens. Such mice, in contrast to untreated mice, demonstrate a decreased response to donor spleen cells les measured in mixed lymphocyte assays) when GVHD is induced by injection of donor spleen cells. This study is further described in Example 11 in the experimental section that follows next.

This invention provides a use for treating Crohn's disease in a subject. In this use selective immune down regulation in the subject to inflamed intestine indicative of Crohn's disease is established by introducing into the subject components, cells, tissues or organs derived from allogeneic sources, and autologous sources, or an immunologically equivalent, as explained elsewhere in this disclosure.

### Reference Example 1 Establishment of Oral Tolerance to Recombinant Adenovirus Antigens

Enteral exposure to foreign antigens has been shown to induce antigen specific tolerance by clonal inactivation of antigen specific T cells or by the induction of regulatory cells secreting factors that suppress the generation of antigen-specific effector cells (Weiner, et al., 1994, Proc. Natl. Acad. Sci. USA 91:10762-10765; Vandenback, et al., J. Immunol. 153:852-9 (1994); and Hirahara, et al., J. Immunol. 154:6238-6245 (1995)). Therefore, in this example, high dose and low dose tolerizing regimens were used and it was demonstrated that induction of oral tolerance to adenoviral antigens can be used to abrogate the host anti-adenoviral immune response in a model system that employs Gunn rats, a strain that lacks hepatic bilirubin uridine-diphosphoglucuronate glucuronosyltransferase-1 (BUGT₁).
***Animals:*** Inbred Gunn and congenic normal Wistar RHA rats were bred and maintained in the Special Animal Core of the Marion Bessin Liver Center of the Albert Einstein College of Medicine. The rats were maintained on standard laboratory chow and kept in 12 hr light/dark cycles.
***Plasmids:*** pJM17 was kindly provided by Dr. F.L.Graham, McMaster University, Hamilton, Canada.
***Generation** of **recombinant adenovirus:*** Two recombinant adenoviruses, Ad-hBUGT₁ and Ad-LacZ expressing human bilirubin-UGT₁ and *E. Coli* b-galactosidase, respectively, were generated as described previously (Takahashi (1996) supra). In brief, transcription units consisting of the promoter and enhancer sequence for the immediate early gene of cytomegalovirus (CMV), the structural region of human BUGT₁ or *E. Coli* b-galactosidase, and the polyadenylation signal from bovine growth hormone, were recombined into the E1 region of human Ad-5 to produce replicationdefective 'first generation" adenoviruses. For large-scale preparation, the recombinant adenoviruses were grown on 293 suspension cells and purified from cell lysates by two consecutive CsCl density gradient centrifugations, and stored in 30% glycerol at -20°C. Virus was dialyzed overnight at 4°C against an isotonic solution containing 135 mM NaCl, 5 mM KCI, 1 mM MgCl₂, 10 mM Tris-HCl, pH 7.4, and 10% glycerol, and sterilized by filtration through 0.45 um filters before use (Horowitz (1990) supra).
***Preparation and administration of viral protein extract:*** The CsCl gradient supernatant, containing major adenoviral structural proteins, mainly fiber, hexon and penton, was collected and the protein concentration was determined (Horwitz, et al., Virology 39: 682-694 (1969); Mazel, et al., Virology 36:126-136 (1968)). Under ether anesthesia, a polyethelene cathether (PE10) was inserted into the stomach through a midline incision. The tube was advanced into the duodenum and affixed to the stomach wall. The other end of the catheter was exteriorized at the dorsal aspect of the neck by subcutaneous tunneling. Each of the rats was kept in a separate cage throughout the study. The protein extracts were introduced through the catheter every other day for 21 days (a total of 11 doses).

**Table 1: Experimental groups:**

| Group | Antigen fed and dose: | Recombinant virus injected on first injection | Recombinant virus injected on second injection |
|---|---|---|---|
| A | adenoviral proteins: 1 mg per rat per day | Ad-hBUGT₁ | Ad-hBUGT |
| B | adenoviral proteins: 1 mg per rat per day | Ad-hBUGT₁ | Ad-LacZ |
| C | bovine serum albumin : 1 mg per rat per day | Ad-hBUGT₁ | (C1) Ad-hBUGT₁ (5 rats) |
| | | | (C2) Ad-LacZ (5 rats) |
| D | None | | Ad-hBUGT₁ Ad-hBUGT₁ |
| E | adenoviral proteins: 50 mg per rat per day (n = 5), or 100 mg per rat per day (n = 5) | Ad-hBUGT₁ | Ad-hBUGT₁ |

***Ad-hBUGT₁ and Ad-LacZ injection Into Gunn rats:*** Five groups of Gunn rats, consisting of 10 animals in each group, were studied. Groups A and B included rats that were fed with adenovirus protein extract at a dose of 1 mg/rat every other day followed by two injections of Ad-hBUGT₁ (5xIO⁹pfu) on days 1 and 98 (Group A), or Ad-hBUGT₁ on day 1 followed by Ad- LacZ on day 98 (Group B). Two groups of rats were used as controls: Group C received bovine serum albumin 1 mg/day and then received viral injections as described for Group A (5 rats, Group C1) or for Group B (5 rats, group C2). Group D did not receive any oral proteins and was injected with Ad-hBUGT₁ similarly to Group A (Table 1). In order to evaluate the mechanism of the tolerance, and distinguished between induction of suppressor cells and clonal inactivation Group E rats were fed with 50mg/day (5 rats), or 100 mg/day (5 rats) of the viral protein extract (high dose regimen), followed by two injections of Ad-hBUGT₁.

### Evaluation of immune tolerance

***Liver histology:*** For evaluation of the degree of hepatic inflammation, liver biopsies were performed one week after the second injection in 2 rats from each of the treated groups and kept in 10% formaldehyde. Paraffin sections were then stained with hematoxylin-eosin according to standard procedures. The sections were graded for hepatic inflammation as follows: Grade 0: normal; Grade 1: mild periportal or focal lobular lymphocytic infiltration; Grade 2: extension of lymphocytic infiltration into the lobules and "piece-meal necrosis"; and Grade 3: disruption of the lobular architecture by "bridging necrosis" and extension of lymphocytic infiltrates from portal to central, portal to portal and central to central zones.
***Serum alanine amino transferase (ALT) levels:*** As a measure of the degree of hepatic inflammation, ALT levels were quantified using a commercially available kit (Sigma, St Louis, MO).
***Neutralizing anti-adenoviral antibodies:*** Anti-adenoviral neutralizing antibodies present in the sera of treated rats were measured on days 28, 78, 112, and 196 in all rats that received Ad-hBUGT₁ injection. 293 cells were seeded at a concentration of 3xIO⁴ well in 96 well plates, and cultured until 90% confluency. Ad-LacZ was diluted in cell culture medium to give 3xIO⁵ pfu /10ml. Serum samples were heat inactivated at 55°C for 30 min. and diluted in medium in twofold steps. 100 ml of each serum dilution was mixed with 5xIO⁵ pfu of the recombinant virus, incubated at 37°C for 90 minutes, and applied to the nearly confluent 293 cells for 10-14 hours. The supernatant containing serum and virus was then replaced by RPMI medium with 10% FCS for 18 hours. Cells were fixed and stained for b-galactosidase expression. In the absence of neutralizing antibodies all of the cells stained blue. The neutralizing antibody titer for each serum sample was reported as the highest dilution at which less than 25% of the cells stained blue.
*Cytotoxic T **lymphocyte** assay.* Two rats from each group were studied on days 28, 78, 112 and 196. Spleens were removed under anesthesia from each of two rats at each time point, and the animals resutured. The organs were gently disrupted using a rubber policeman. Red blood cells were removed using lysis buffer containing 0.17M NH₄Cl at pH 7.4 (1 ml/spleen) for 2 minutes. Lymphocytes were spun down and plated at 5x10⁷cells per 5 ml in RPMI medium with 10% FCS. Cells were then restimulated with the recombinant adenovirus Ad-hBUGT₁ (1-10 pfu/cell) for 4-5 days. Adenovirus infected primary hepatocytes, harvested by collagenase perfusion of the liver (Seglen, Methods in Cell Biology, (1976)), were used as target cells for the effector lymphocytes and were plated on collagen coated 6 well plates in Chee's medium (2x10⁶ cells/well). Stimulated effector cells were harvested, counted and added to the primary hepatocyte cultures at a ratio of 50-100:1 and incubated at 37°C for 5 hours. Hepatic cell lysis was measured by collecting the medium and measuring alanine aminotransferase (ALT) levels using a commercially available kit (Sigma, St Louis MD) with the following modifications: the ratio between reagent and test medium was changed from 10:1 to 1:1, and the reaction time before the first spectrophotometric reading was 90 seconds, followed by a reading every 30 seconds up to 5 minutes. ALT levels were then calculated according to the manufacturers' formula and expressed in international units. Background ALT levels were determined by measurements of the ALT levels in the supernatants of dishes containing adenovirally infected hepatocytes and lymphocytes from naive rats. CTL activity was expressed in IU of ALT averaged from 6 wells after subtraction of background levels.

### RESULTS

### Evaluation of immune tolerance

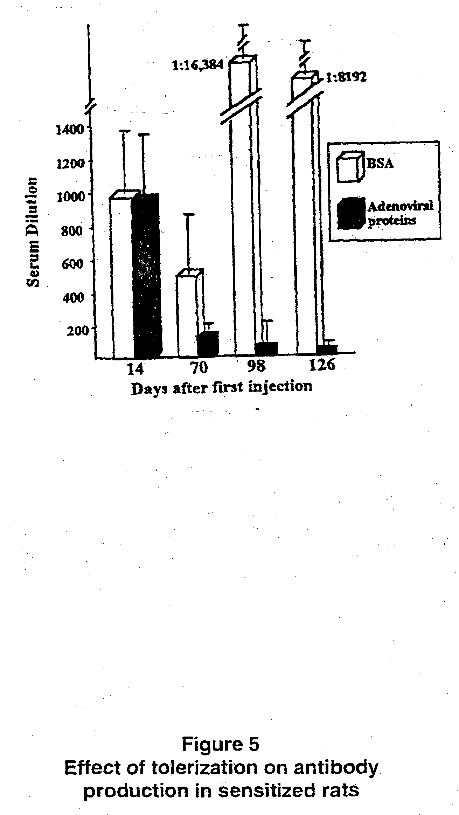

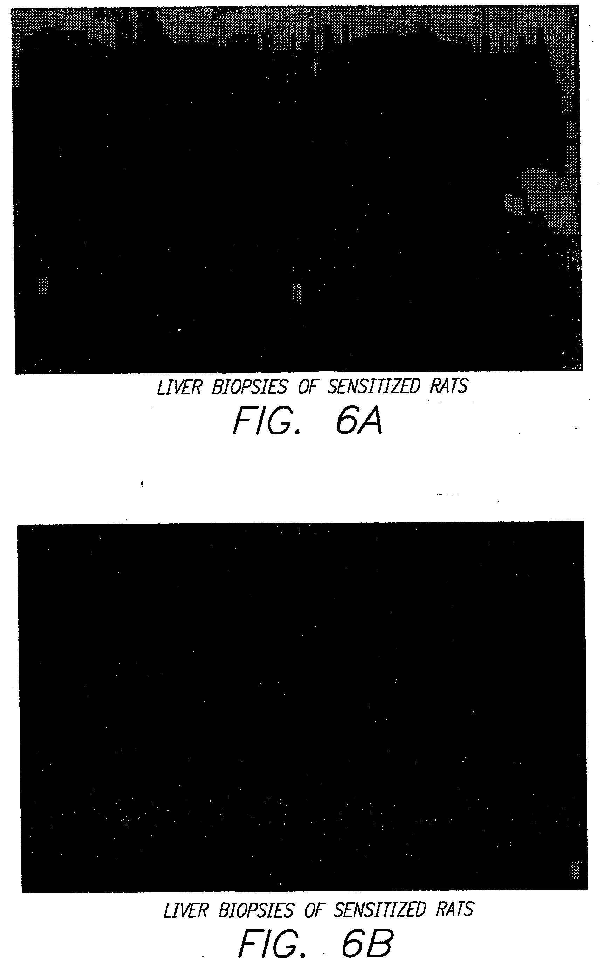

***Liver histology.*** Liver biopsies from two rats in each group examined 24-72 h after the second injection showed minimal or no periportal or lobular lymphocytic infiltration in recipients that were tolerized by enteral administration of adenoviral proteins (group A). In contrast, a severe inflammatory reaction (grade 3) was observed in liver specimens taken from rats that were given BSA or no protein prior to the injection of the virus (groups C, D) and rats that received high doses of adenoviral antigens (group E) (not shown).
***Serum ALT levels:*** In the group A rats that were tolerized with adenoviral proteins, serum ALT levels increased only minimally after each of the three injections (96-110 IU; normal levels before any manipulation were 60-75 IU). In groups that received BSA or no protein (C and D), ALT levels increased to 168 IU after the first Injection, and to 212 IU after the second injection.
***Neutralizing antibodies*:** After injection of Ad-hBUGT₁ in rats that had received BSA or no proteins (Groups C, and D, respectively) high titer (> 1:2816) antibodies appeared during the first month. In contrast, in the tolerized rats (Group A), neutralizing antibodies were undetectable in 80% of the recipients. The remainder exhibited low titers of the antibody (< 1: 16) (Fig. 5). Rats that developed the low titer antibodies had similar hypobilirubinemic responses to the second injections of Ad-hBUGT₁ as did the rats that had no detectable antibodies.
***Cytotoxic T lymphocyte response:*** Cytotoxic T cells were tested against adenovirus infected rat hepatocytes four times throughout the study. Measurement of the amount of ALT released from the hepatocyte targets into the media was used to assess the CTL response. ALT levels in the media were below 80 IU in all tolerized recipients (groups A and B), but exceeded 450 IU in non-tolerized rats (groups B and C) (Fig. 6).
***Effect of antigen dose:*** To evaluate the relationship between the dose of adenoviral proteins and induction of tolerance, 10 rats in group E were fed with the viral proteins at higher doses (50-100 mg/day). In these rats serum bilirubin levels and HPLC analysis of pigments excreted in bile indicated that the second recombinant adenoviral injection failed to achieve gene expression or a metabolic effect. The anti-adenovirus immune response in this group was similar to that in rats that were administered BSA or no protein at all (groups C and D). Thus although no evidence for tolerance was observed using higher doses of the antigen, the administration of low dose feeding of adenoviral proteins markedly inhibited both humoral and cellular host immune response to the recombinant adenovirus containing the human BUGT₁ gene.
High dose feeding, which has been shown to induce anergy or deletion of antigen-reactive T cells, was found to be ineffective, In contrast, oral tolerization with low dose feeding of adenoviral protein extracts, markedly inhibited both the humoral and cellular host immune response to the recombinant adenovirus containing the human BUGT₁ gene.

### Reference Examples 2 Oral Tolerization to Recombinant Adenovirus Prolongs Expression Time and Permits Readministration

The animals used in Example 1 were also used to evaluate the effect of an oral tolerization regime on the length of expression from recombinant adenoviruses.

### Assessment of transgene expression.

***b-Galactosidase expression:*** Gunn rats from groups that received AdhBUGTi as the first injection and Ad-LacZ as the second injection with (group B) or without (group C2) previous administration of adenoviral proteins underwent liver biopsies. Specimens were frozen in Tissue Freezing Medium (Triangle Biomedical Sciences, Durham, NC), in a dry ice cooled methyl butane bath. Frozen Cryostat sections (10 um) were fixed for 5 minutes at room temperature in freshly prepared 1 % glutaraldehyde in PBS. β-Galactosidase activity was detected by immersing the section into 5-bromo-4-chloro-3-indol-b-galactopyranoside (X-Gal) staining solution (5mM K₄FeCN, 5mM K₃FeCN, ImM MgCl₂, containing 1 mg of X-Gal per ml) for 8-15 hours at 37°C. Sections were briefly counterstained with eosin, then dehydrated and mounted.
***DNA analysis using PCR:.*** To detect the presence of the human BUGT₁ gene in the host liver, DNA was extracted from RNase treated tissue homogenates. Two rats from each of the experimental groups A and E, and the control groups C1 and D, were tested 3 days after the second Ad-hBUGT₁ injection. DNA was subjected to amplification by polymerase chain reaction (PCR) using primers (sense:
   5'AAGGAAAGGGTCCGTCAGCA 3' from nt 141 to nt 160, antisense: 5'CCAGCAGCTGCAGCAGAGG 3' from nt 441 to nt 462) designed to amplify a 321-bp segment of the the unique exon 1 (exon 1*1) of the human BUGT₁ gene. PCR amplification was performed using the following protocol: 94°C for 30 sec, 58°C for 30 sec, and 72°C for 1 min x 30 cycles.
***Expression of human-BUGT, protein:*** For determination of the expression of hBUGT₁ liver specimens were taken from two rats in experimental Group A and control Group C1 five days after the second viral injection. Tissue homogenates (200 mg/ml) were prepared in 0.25 M sucrase/10 mM Tris-HC1, pH 7.4 using a glass homogenizer fitted with a motor-driven teflon pestle. For immunoblot analysis, proteins (100 mg/lane) were resolved by electrophoresis on SDS-polyacrylamide (7.5%) gels and electroblotted to nitrocellulose membranes. The membranes were probed with a monoclonal antibody WP1 directed at the common carboxyterminal domains of UGT isoforms expressed by hBUGT₁, followed by peroxidase conjugated goat anti-mouse IgG F'ab fragment second antibody (Sigma, St. Louis, MO) and substrate (Peters, et al., Gastroenterology 93:162-169 (1987); Towbin, et al., Proc. Natl. Acad. Sci. USA 76:4350-4354 (1979)). Equal protein loading in all lanes was assured by performing the electroporesis on an identical SDS-polyacrylamide gel and staining the protein bands with Coomassie brilliant blue.
***Assay for BUGT₁ activity towards bilirubin:*** The enzyme assay was performed on homogenates of liver specimens from two rats from each experimental group that received Ad-hBUGT₁ injection (A, E, C1 and D), 20 days after the first and second injection, and from all other rats at the termination of the experiments. The assay method was as previously described, using 80 mM bilirubin as the aglycone (Trotman, et al., Anal Biochem 121:176-180 (1982); Roy Chowdhury, et al., Hepatology 1:622-627 (1981)).
***Determination of serum bilirubin levels:*** Serum bilirubin levels were measured according to Jendrasik and Grof in all groups every 10-14 days throughout the study period (Trotman (1992) supra).
***Bile pigment analysis:*** For definitive demonstration of bilirubin glucuronidation in selected rats, bile was collected through a polyethylene bile duct cannula and bilirubin glucuronide excreted in bile was analyzed by HPLC using a uBondapak C-18 column (Millipore-Waters, Milford, MA) as described previously (Roy Chowdhury (1982) supra). Bile was analysed in two rats from experimental groups that received adenoviral proteins at 1 mg/day (group A), 50 mg/day or 100 mg/day (group E) BSA 1 mg/day (groups C1) or no protein at all (D), 20 days after the first and second injections of the recombinant adenovirus. All other rats had bile pigment analysis at the termination of the experiments.

### RESULTS

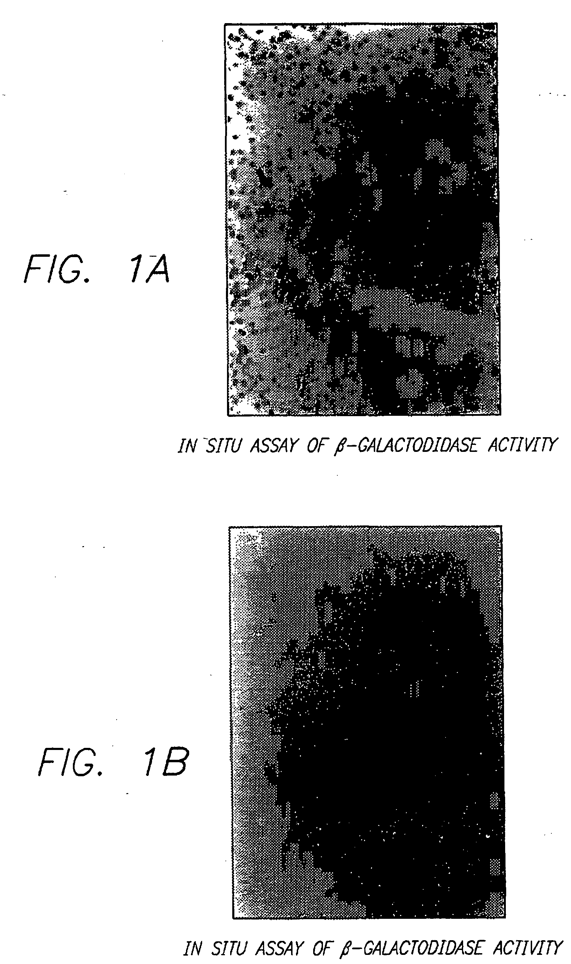

**Expression of b-galactosidase activity:** For histochemical staining, liver biopsies were performed, 7 days after Ad-LacZ injection from liver specimens of Gunn rats that received AdhBUGT₁ as the first injection and Ad-LacZ as the second injection with (group B) or without (group C2) prior administration of adenoviral proteins. Biopsies were performed on two rats in each group. Histochemical staining of cryostat sections (10 mm) showed that the great majority of hepatocytes stained positive for b-galactosidase activity after the injection in rats that had been administered the adenoviral proteins (group B), while only 5% of hepatocytes stained positive, in livers from rats that were given BSA (group C2) (Fig. 1) .

### Expression of h-BUGT gene after recombinant adenoviral injection into Gunn rats.

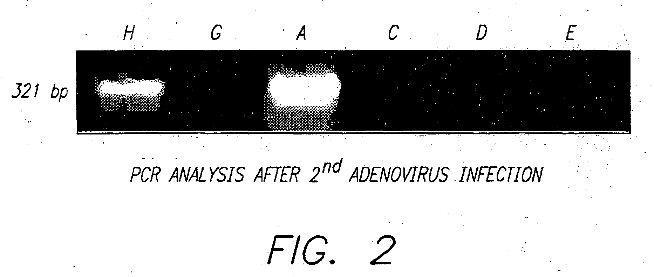

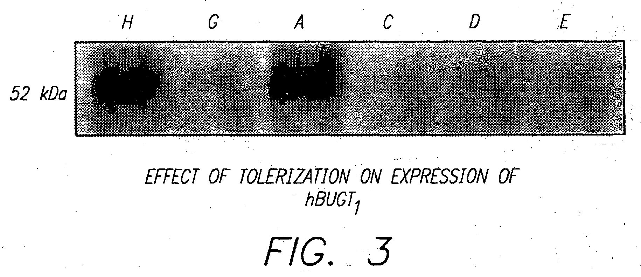

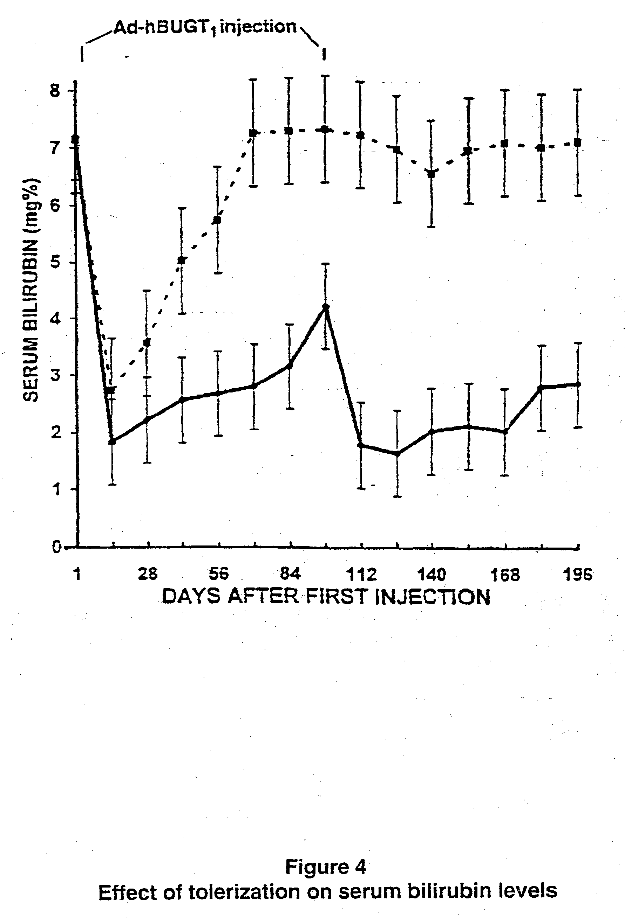

***DNA analysis using PCR:*** Presence of hBUGT₁ DNA in the liver of Gunn rats that received Ad-hBUGT₁ with (group A) or without (groups C1 and D) without prior enteral administration of adenoviral proteins was evaluated by PCR after the second AdhBUGT₁ injection. A DNA fragment of 321 bp was seen only in rats from group A, while both control groups C and D, were negative. Normal human liver and liver from an untreated Gunn rat were used as positive and negative controls, respectively (Figure 2).
***Expression of human-BUGT₁ protein:*** Liver specimens were collected from two rats in groups A and C1, 5 days after the second Ad-hBUGT₁ injection. Immunoreactive 52 kDa bands, corresponding to hBUGT₁ were observed in Gunn rats that were given two injections of Ad-hBUGT₁ after the administration of adenoviral antigens (group A) but not in the group that received the virus injections after enteral administration of BSA (group C) (Fig. 3). Normal human liver and untreated Gun rat livers were used as positive and negative controls respectively.
***BUGT₁ activity in vitro:*** UGT activity toward bilirubin was undetectable in untreated Gunn rats. In homogenates of normal human specimens obtained from cadaver donor organs, the BUGT₁ activity was 78 ± 26 nmol/mg liver weight/min; (mean ± SEM, n = 6). In liver homogenates from two rats that received Ad-hBUGT₁ injections after enteral administration of adenoviral proteins (group A), bilirubinUGT activity was 80 and 85 nmol/mg liver wet weight/min, 20 days after the first Ad-hBUGT₁ injection, and was 88 ± 20 nmol/mg liver wet weight/min (mean ± SEM, n=5) 20 days after the second injection. In the BSA-fed rats, (groups C1 and D), bilirubin-UGT activity was undetectable after the second injection of ad-hBUGT₁.
***Serum bilirubin levels:*** Bilirubin levels were measured every 10-14 days. A marked decrease in bilirubin levels occurred after each Ad-hBUGT₁ injection in Gunn rats that were tolerized by the administration of adenoviral proteins (group A), with levels reaching as low as 1.83 , and 1.78 mg/dl after the first and second injections, respectively (Fig. 4). Bilirubin levels remained low for over three months after each injection, and then increased gradually. In contrast, in BSA-fed Gunn rats (Groups C and D) the first Ad-hBUGT₁ injection reduced serum bilirubin levels to 2.73 mg/dl for only 4 weeks, followed by a progressive increase to preinjection levels. Subsequent Ad-hBUGT₁ injections had no effect on serum bilirubin concentrations in these groups.
***Bile pigments analysis:*** HPLC analysis of bile collected from two rats from AdhBUGT₁-treated rats (groups A, C1, D and E) 20 days after the first injection of AdhBUGT₁ showed excretion of bilirubin mono- and diglucuronide. The two glucuronides accounted for more than 95% of the bile pigments, less than 5% being unconjugated bilirubin. This profile was similar to that seen in normal Wistar rats. A similar pattern was seen in rats tolerized by enteral administration of 1 mg/day adenoviral proteins (group A) 20 days after the second Ad-hBUGT₁ injection. In rats that received BSA (group C), no protein (group D) or high doses of adenoviral proteins (50-100 mg/day, group E), bile pigment analysis after the second Ad-hBUGT₁ injection did not show significant amounts of conjugated bilirubin in the bile. Gunn rats injected with Ad-LacZ did not excrete bilirubin glucuronides in bile. Chromatographic profiles in bile from these rats resembled that from untreated Gunn rats.

### DISCUSSION

Oral tolerization prolonged the transgene expression, as shown by the longer duration of the hypobilirubinemic effect. However, the effect was not permanent, as indicated by the gradual increase of serum bilirubin levels between day 14 and 98 after the first Ad-hBUGT₁ injection. A similar decay of transgene effect was seen after induction central tolerance to recombinant adenoviruses induced by injection of the virus during the newborn period (Takahashi (1996) supra). The decline of transgene effect seems to have resulted from the degradation of the episomal adenoviral DNA, rather than the loss of tolerance, because there was no antiviral CTL activity in the host during this period.

In conclusion, this example shows the potency of low dose oral viral antigen administration in down-regulating the antiviral immune response. In addition, this example demonstrates that after the activity of the desired gene product is diminished, the recombinant adenovirus can be re-administered without an immune reaction. This method is useful in clinical practice in order to tolerize the host to a useful recombinant adenovirus, and opens the possibility of providing effective long-term gene therapy for inherited metabolic diseases using these vectors.

### Reference Example 3 Oral Tolerization to Recombinant Adenovirus is Stable

To determine whether oral tolerization results in long-term tolerance, Gunn rats were tolerized by oral administration of adenoviral proteins as described in example 1. Eight months after the tolerization, the rats were injected with Ad-hBUGT₁. This did hot result in either humoral or CTL response. These results indicate that even in the absence of the presence of the tolerizing antigens, the tolerization is long-lived.

### Reference Example 4 Establishment of Oral Tolerization to Pre-Existing Immunoreaction

The previous examples have demonstated that long-term adenovirus-directed gene therapy, can be achieved by tolerizing the host specifically to antigens of the recombinant adenovirus by oral administration of adenoviral antigens. This permits long-term transgene expression without systemic immunosuppression. Some serotypes of adenoviruses, including adenovirus type 5, the serotype in which most gene therapy vectors have been constructed, commonly infect humans. Therefore, many adult humans have preexisting neutralizing antibodies and cytotoxic lymphocytes against adenoviruses, which would pose an obstacle to the clinical application of these vectors (Weiner (1994) supra; Vandenback (1994) supra).

The present example demonstrates that oral tolerization, in addition to preventing the appearance of host immune response, can also reduce preexisting antiadenoviral antibody titers and cytotoxic lymphocyte response. The results demonstrate, for the first time, that by enteral administration of the major adenoviral structural proteins into preimmunized rats it is possible to reduce the preexisting antiadenoviral immune response to a point at which it possible to express the transgene by intravenous injection of a recombinant adenovirus.

Details of the protocols for the tolerization viral administration and analysis of immunological effects were as described for Example 1. Details of the protocols for assessment of gene expression were as described for Example 2.
**Ad-hBUGT₁ injection into Gunn rats:** Three groups of Gunn rats, two consisting of 10 animals in each (A and C), and one with five rats (B), were studied (Table 1). All rats were injected with the recombinant virus Ad-HBUGT (3x10⁹ pfu/rat) and the induction of high titers of anti-adenovirus neutralizing antibodies was verified as described below. Groups A rats were fed with 10 doses of adenoviral-protein extracts (1 mg every other day) starting on day 40 after the first adenovirus injection. Rats in Group B were fed with 5 doses of the adenoviral proteins starting on day 10. Group C rats (control) received 10 doses of bovine serum albumin (1 mg every other day) starting on day 40. Rats in all groups received a second injection of Ad-hBUGT₁ (5 x 10⁹ pfu) on day 72.

**Table 2: Experimental groups:**

| **Group** | **(n)** | **Antigen fed and dose** | **Number of doses** | **Days of feeding after first virus injection)** |
|---|---|---|---|---|
| **A** | 10 | Adenoviral proteins 1 mg per rat daily | 10 | 40-58 |
| **B** | 5 | Adenoviral proteins 1 mg per rat daily | 5 | 10 - 18 |
| **C** | 10 | 1 mg bovine serum albumin per rat per day | 10 | 40 - 58 |

### Assessment of transgene expression.

***DNA analysis using PCR:*** To detect the presence of the human BUGT₁ gene in the host liver, DNA was extracted from RNase-treated tissue homogenates as described previously in Example 2. Two rats from each group were tested 5 days after the second Ad-hBUGT₁ injection. DNA was subjected to amplification by polymerase chain reaction as described in Example 2
***Expression of human-BUGT₁ protein:*** For determination of the expression of hBUGT₁, liver specimens were taken from two rats in experimental Groups A and B, and control Group C five days after the second viral injection. Tissue homogenates were processed and analyzed as described in Example 2.
***Determination of serum bilirubin levels**:* Serum bilirubin levels were measured every 10-14 days throughout the study period as described in Example 2.
***Bile pigment analysis:*** For definitive demonstration of bilirubin glucuronidation in selected rats, bile was collected and analyzed as described in Example 2. Bile was analyzed in two rats from each group, 14 days after the second injection.

### Evaluation of immune tolerance.

***Antiadenovirus antibodies by enzyme-linked immunosorbent assay (ELISA):*** Detection of antiadenoviral antibodies by ELISA was performed by coating 96 well plates with 1x10⁸ particles per well of Ad-hBUGT in PBS at 4°C overnight. The wells were washed five times with 10 mM sodium phosphate containing 150 mM NaCl (PBS) and 1% Tween-20, blocked with 3% BSA in PBS, washed again and incubated for 2 hours with serial dilutions of the sera (in 1 % BSA) at 37°C. IgG antibody levels were measured after 0.1 M mercapthoethanol incubation of the sera for 1 hour at 37°C. The wells were washed and incubated with 100ml of 1:1000 dilution of alkaline phosphatase-conjugated goat anti-rat IgG (Bethyl Laboratories, Montgomery, TX), for 2 hour at 37°C. After washing, the wells were incubated with substrate (104 Phosphate Substrate, Sigma Diagnostics, St Louis), and read at 405 nm in an ELISA reader. Two negative control sera from naive Gunn rats, were included in each plate. End point titers were expressed as the reciprocal of the highest dilution that produced an absorbance at least two-fold greater than that observed with negative controls. Sera of rats from all groups were tested on days 0, 14, 70, 98 and 126, after the first injection.
***Liver histology:*** For evaluation of the degree of hepatic inflammation, 10% formaldehyde-fixed liver biopsies were performed one week after the second injection in 2 rats from each group. Paraffin sections were stained with hematoxylin-eosin according to standard procedures. The sections were graded for hepatic inflammation as follows: Grade 0: normal; Grade 1: mild periportal or focal lobular lymphocytic infiltration; Grade 2: extension of lymphocytic infiltration into the lobules and "piecemeal necrosis"; and Grade 3: disruption of the lobular architecture by "bridging necrosis" and extension of lymphocytic infiltrates from portal to central, portal to portal and central to central zones.
***Cytotoxic lymphocyte response:*** Two rats from each group were studied on days 50 and 98. Spleens were removed under anesthesia from each of two rats at each time point, and the animals resutured. Subsequent analysis was as described in Example

### RESULTS

### The effect of oral tolerization.

***Antiadenovirus antibodies:*** Serum IgG anti-adenovirus antibodies were examined by ELISA on days 0, 14, 70, 98 and 126 after the first injection, in all rats from groups A, B, and C. Anti-adenovirus antibodies appeared in all three groups after the first injection, with titers rising to a peak of 1:2¹⁰ at day 14 (Fig 2). However after enteral administration of 10 doses of adenoviral antigens (Group A), the antibody titers decreased to 1:2⁷ at day 70 (Fig 5, solid bar). In the BSA-treated controls (Group C), there was only a slight decrease (1:2⁹) in the anti-adenovirus antibody-titers (Fig. 5, open bars). Following the second injection of Ad-hBUGT₁ on day 72 after the first injection, there was a boosting of antibody titers (1 :2¹⁴) in the control group. In contrast, the antibody titers progressively decreased in the orally tolerized rats (Group A, Fig 5, solid bars) despite the second injection of the recombinant adenovirus. Similar results were seen in the tolerized Group B, in which only 5 doses of the adenoviral proteins were administered between day 10 and 18 after the first adenovirus injection (not shown in Fig. 5). Twenty six and 54 days after the second adenovirus injection, the difference in antibody titers between the tolerized and non-tolerized groups were statistically significant (p < 0.005, by Student's T test)
***Cytotoxic lymphocyte response:*** Cytotoxic T cells against rat hepatocytes infected with adenoviruses were tested twice (day 50 and day 98 after the first Ad-hBUGT₁ injection) during the study, in two rats from Groups A and C. ALT activity released in the media from the hepatocyte targets was used to quantify the CTL response. ALT levels in the media were 449 and 409 IU in Group A, and 421 and 531 IU in Group C 50 days after the first virus injection. Twenty six days after oral tolerization, ALT activity released in the media decreased to 166 and 142 IU in Group A, even though these rats had received a second dose of Ad-hBUGT₁ on day 72. In the BSA-treated controls (Group C) the CTL activity, as reflected by ALT activity in the media continued to be high (399 and 476 IU).
***Liver histology:*** Liver biopsies from two rats in each group examined 24-72 h after the second injection showed minimal or no periportal or lobular lymphocytic infiltration in group A. In contrast, a severe inflammatory reaction (grade 3) was observed in liver specimens taken from group C (Fig. 6).

### Expression of H-BUGT gene after the Injection of Ad-hBUGT₁ into Gunn rats.

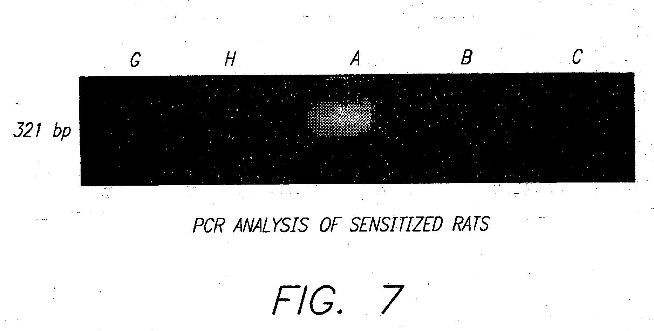

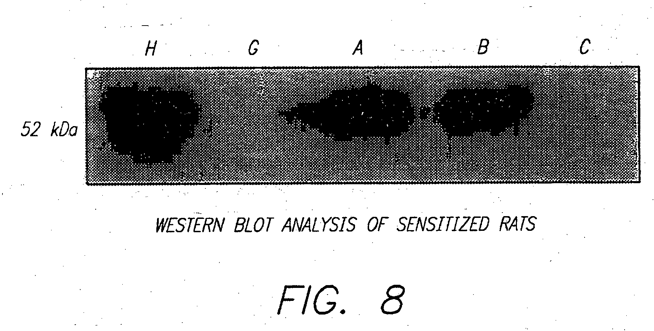

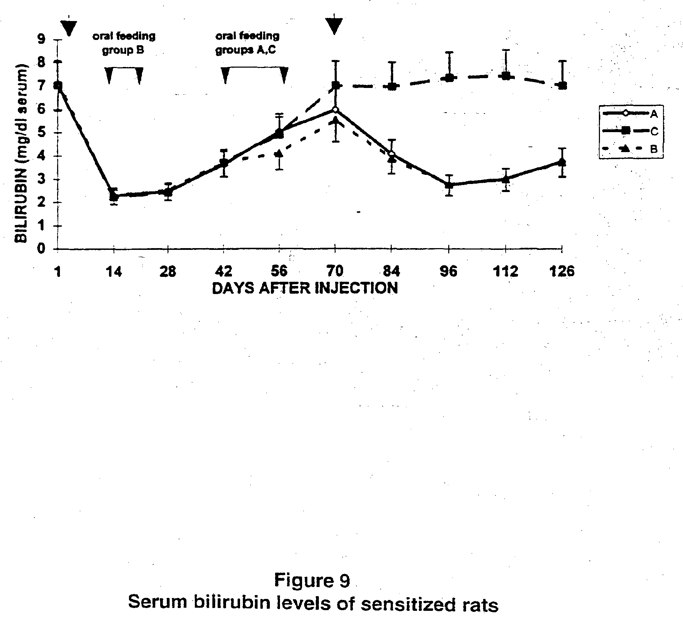

***DNA analysis using PCR:*** Presence of human BUGT₁ DNA in the liver of Gunn rats from groups A and B was tested by PCR after the second AdhBUGT₁ injection. The expected 321-bp amplicon was seen only in rats from groups A and B, while control group C was negative. Normal human liver and liver from an untreated Gunn rat were used as positive and negative controls, respectively (Fig 7).
***Expression of human-BUGT₁ protein:*** Liver specimens were collected from two rats in groups A, B, and C, 5 days after the second Ad-hBUGT₁ injection. Immunoreactive 52 kDa bands, corresponding to hBUGT₁, were observed in the treated Gunn rats in groups A and B following the second injection, but not in rats from the control group C (Fig. 8). Normal human liver and untreated Gun rat livers were used as positive and negative controls respectively.
***Serum bilirubin levels:*** Bilirubin levels were measured every 10-14 days. A marked decrease in bilirubin levels occurred after each Ad-hBUGT₁ injection in groups A and B, with levels reaching as low as 2.2, and 2.78 mg/dl after the first and second injections, respectively (Fig. 9). In contrast, in untolerized Gunn rats (Group C), the second Ad-hBUGT₁ injection had no effect on serum bilirubin concentrations.
***Bile pigment analysis:*** HPLC analysis of bile collected from two rats in groups A, B and C, twenty days after the second injection of Ad-hBUGT₁ showed excretion of bilirubin monogluronide and diglucuronide in the bile collected from group A and B. The two glucuronides accounted for more than 95% of the bile pigments, less than 5% being unconjugated bilirubin. This profile is similar to that seen in normal Wistar rats. In group C, bile pigment analysis following the second injection showed no significant conjugated brain excretion detected in the bile.
This example demonstrates that the level of preexisting antibodies against adenoviruses can be suppressed by oral instillation of the major viral proteins into preimmunized rats. This procedure made it possible to readminister the virus with repeated rounds of transgene expression. The reduction of antibody titers occurs slowly because of the relatively long half-life of immunoglobulins, but importantly, the antibody levels continued to decrease in the tolerized group despite the second injection of Ad-hBUGT₁. The findings indicate that the presence of antibodies in titers of up to 1:2⁷ does not impede gene transfer using adenoviral vectors. The lack of a detectable metabolic effect after the second administration of the virus in the non-tolerized (BSA-treated) group suggests that the strong secondary humoral or CTL response that resulted from the second injection may be responsible for attenuating the transgene expression by clearing the recombinant virus or virally infected hepatocytes.

### Reference Example 5 Oral Tolerization to Recombinant Adenovirus is Transferable

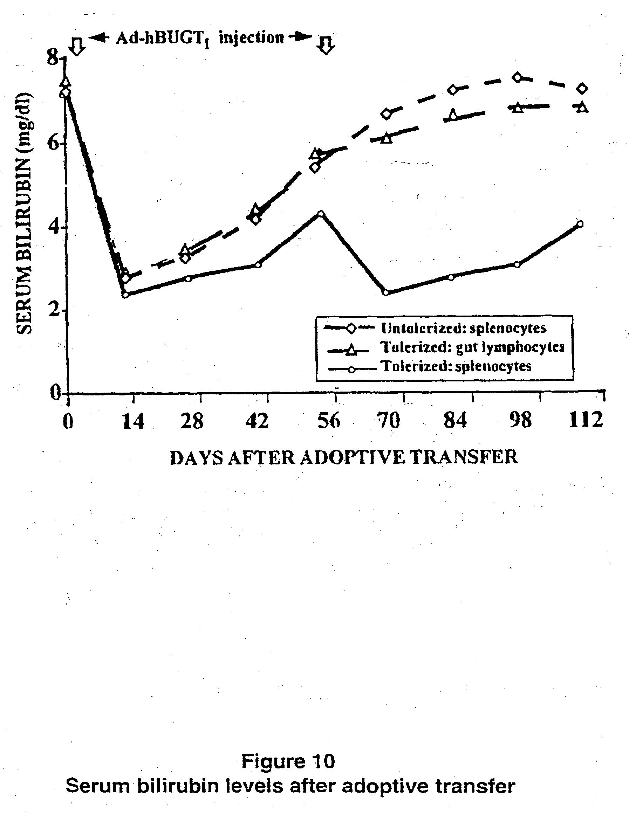

Although the preceding examples have demonstrated the induction of tolerance to recombinant Adenoviruses, there may be circumstances where the tolerization cannot be done in the subject. For instance it has previously shown that the presence of inmmunosuppressive drugs such as cyclosporin A may prevented the induction of oral tolerance (Fukushima, A. Whitcup, S.M., Nussenblatt, R.B., and Gery, I. "In Oral Tolerance; Mechanisms and Applications" (1996) 376-378, H.L. Weiner and L.F. Mayer (eds.) The New York Acadamy of Sciences, New York, N.Y., incorporated herein by reference). Presumably other circumstances that have shut down some of the immune system could also abrogate induction. Under such circumstances it may be useful to induce oral tolerization in one subject (donor) and then transfer this tolerance to a second subject (recipient).
***Adoptive transfer of tolerance:*** To determine whether the intestinal wall or splenic lymphocytes from the tolerized rats are capable of producing tolerance upon transplantation into naive rats, donor rats from groups A and C1 (2 rats from each group) from Examples 1 and 2 were killed at the end of the experiment and single suspensions of lymphocytes derived from the spleen or the small intestine were prepared as described previously for Cytotoxic T lymphocyte assays. The cells were resuspended in PBS immediately before transplantation. Recipients rats were sublethally irradiated with 600 rad total body irradiation, 24 hr prior to intravenous injection of 5x10⁷ - 1x10⁸ donor cells in 0. 5 ml PBS. A total of eight rats were studied, four received the cells from group A donor rats, and four from group C rats (in each group, 2 rats received donor splenocytes, 5x10⁸ cells, and two received donor gut wall lymphocytes, 5x10⁷ cells). All rats were injected with Ad-hBUGT₁ twice, one day after cell transplantation and 98 days later. Serum bilirubin levels, pigments excreted in bile and anti-adenoviral cellular and humoral immune responses were determined as described before.
***Evaluation of Adoptive transfer of tolerance:*** Levels of serum bilirubin in this example are shown in Figure 10. Adoptive transfer of the tolerance was seen only in the two rats receiving the splenocytes from group A. Following AdhBUGT₁ administration, these rats showed a metabolic effect similar to that observed in the tolerized rats from group A described in Example 2. Moreover, one of these rats did not develop anti adenovirus antibodies and the other mounted only a low titer antibody response. In contrast, when lymphocytes from untolerized donors or lymphocytes from the gut wall of tolerized donors were used, adoptive transfer of the tolerance did not occur and serum bilirubin returned to levels seen prior to administration of the recombinant adenovirus. Upon a second injection of the virus, these rats failed to show metabolic evidence of BUGT₁ expression. In the recipients of cells from group C rats, all the rats developed a marked anti-adenoviral humoral and cellular immune response, and all lost the transgene effect within 6 weeks of the injection. This example demonstrates that the induction of oral tolerance in one subject is a state that can be transferred into a second subject.

### Reference Example 6 Oral tolerization to Recombinant Adenovirus in rabbits and demonstration that tolerance is also established to a non-native transgene

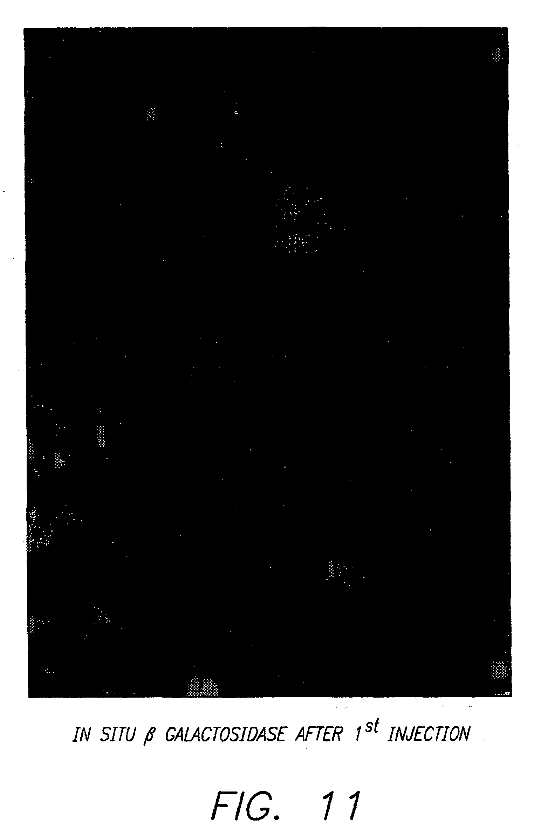

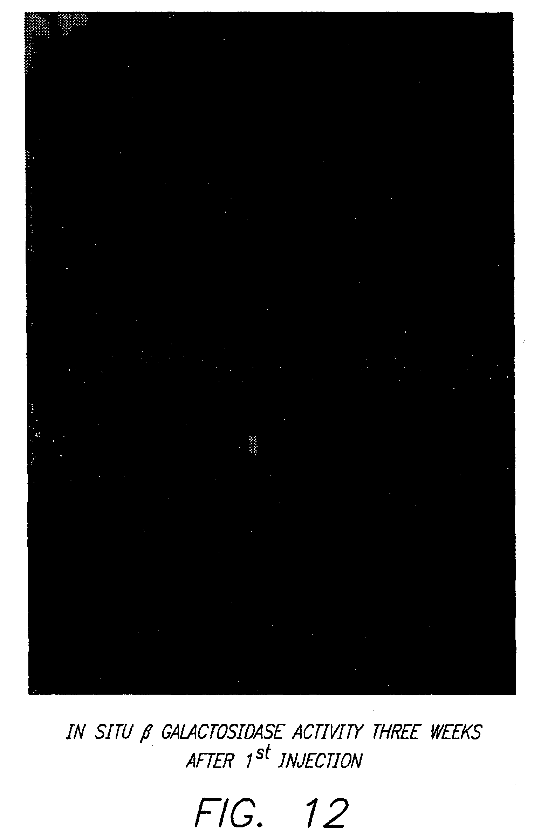

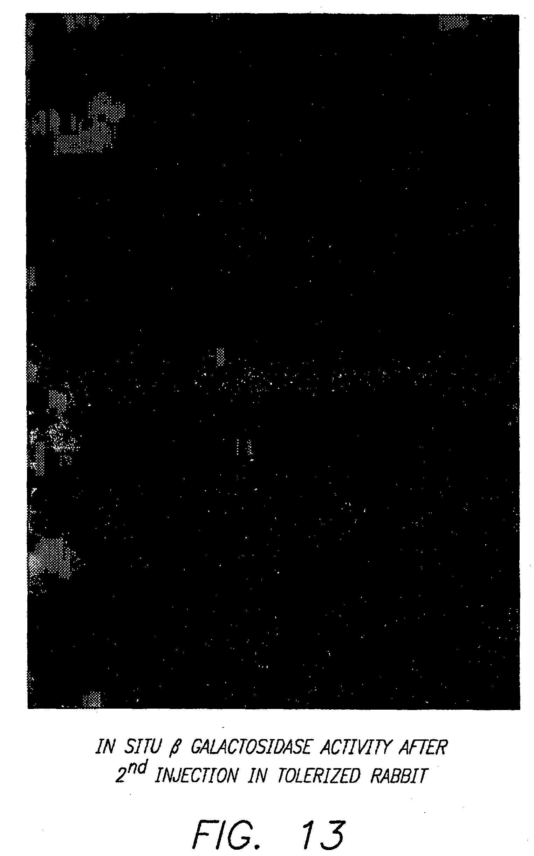

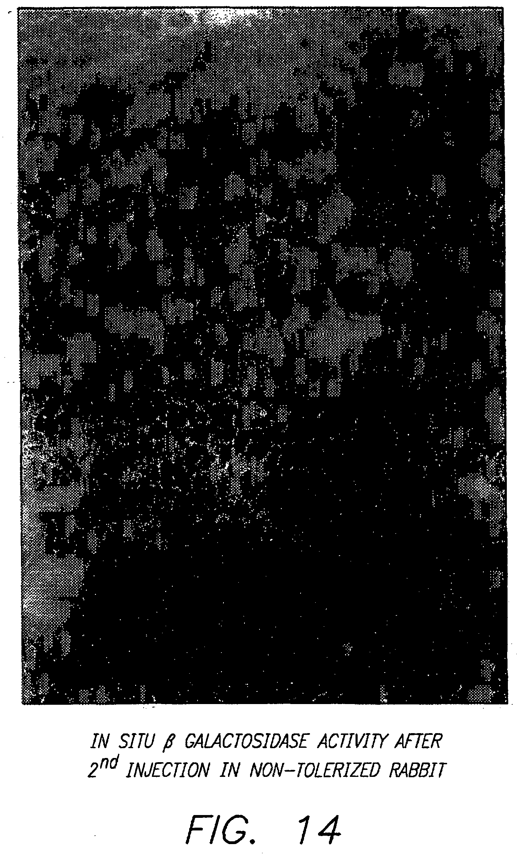

In the previous examples, oral tolerization has provided a stable expression of a recombinant Adenovirus and the tolerization was sufficient to enable expression after a readministration of this Adenovirus vector. However, these examples were performed using rats as subjects and the gene being expressed (BUGT₁) although derived from a human source may be sufficiently similar to the the native product that there may not have been an elicitation of an immune reaction to the transgene. The absence of BUGT₁ gene expression after the second administration may have been a reaction to the vector alone. The following example differs from the previous example in that rabbits are used as the subjects and the gene used in the first and second recombinant Adenovirus administrations is *lacZ* which is derived from *E. coli.*
Details of the methods used for induction and evaluation of oral tolerance are as described for the previous examples with the Gunn rats with the following exceptions:
a) the rabbits were fed antigens orally rather than using intubation
b) Due to their larger size, the amount of antigen used for tolerization was increased proportionally (10 mg instead of 1 mg)
***Evaluation of oral tolerance;*** Figure 11 shows that there is a high level of expression of the lacZ gene after introduction into rabbit hepatocytes by adenovirus. As seen in the rat system, this is a transient effect and most activity has disappeared after three weeks (Figure 12). When the rabbit is tolerized by oral administration of the Ad-hBUGT₁ vector, a second injection with the recombinant Adenovirus allows efficient expression of the lacZ gene (Figure 13). On the other hand, without this tolerization, a second injection is incapable of expressing any significant levels of β-galactosidase activity (Figure 14). This example demonstrates that the present invention is not restricted to rats alone and works efficaciously in other animals. Furthermore, the high level of expression of *lacZ* after the second administration of recombinant adenovirus demonstrated that there was tolerization to an enzyme that is not native to the subject.

### Reference Example 7

### Example: Delivery of Protein Molecules to Cells

Bovine Serum Albumin was labeled by conjugation with both biotin and fluorescein (BSA-BF) or with fluourescein alone (BSA-F) by the following method:
BSA-BF: BSA (68 mg) was dissolved in 4.8 ml of 0.2M borate buffer, pH 9.0. Biotin-21-NHS ester (4 umoles) in 2 ml of DMF was added dropwise, and the mixture was incubated at room temperature for 2 hours. Fluorescein isothiocyanate (2 umoles) in 200 ul DMF was added and the mixture was incubated overnight at room temperature. The mixture was evaporated to dryness in a rotary evaporator and then redissolved in 5 ml HzO and applied to a G-25 column equilibrated with 50 mM Tris buffer, pH 8.0. The fluoresceinated fractions were collected and stored at O°C.
BSA-F: This was prepared by the above procedure except that the reaction with biotin 21 -NHS was omitted.
U937 cells were grown to approximately 10⁶/ ml and centrifuged, washed in growth medium (RPMI), suspended in 3.5 ml of RPMI, and 0.8 ml was placed in four 35 mm wells. BSA-BF and BSA-F, each at a concentration of 20 mg/ml, were added to the four wells together with 200 ul of RPMI as follows:
1) 20 ul BSA-F
2) 50 ul BSA-F
3) 20 ul BSA-BF
4) 50 ul BSA-BF.
The cell suspensions were incubated overnight at 37'C. One ml of cell suspension was placed in 2.0 ml of RPMI and centrifuged at 1000 rpm for 5 minutes. The supernatants were decanted and the cells were resuspended in 200 ul of RPMI. Samples of each cell suspension were placed on a microscope slide and examined using a fluorescent microscope. The following was observed:
1) 20 ul BSA-F: Against a slight background of fluorescense, fewer than 5% of the cells displayed any fluorescense,
2) 50 ul BSA-F: Against a slight background of fluorescense, fewer than 5% of the cells displayed any fluorescense,
3) 20 ul BSA-BF: Approximately 40% of the cells displayed an intense fluorescense,
4) 50 ul BSA-BF:. Approximately 40% of the cells displayed an intense fluorescense.
Evidence that the biotinylated BSA entered cells was provided by observations of cells that were undergoing mitosis as indicated by the presence of two nuclei in a single cell. Such cells displayed a bright fluorescense that was confined to the nuclei.

### Reference Example 8 TGF-b₁ levels as a marker for oral tolerization

The following example demonstrates the method for establishing that tolerization has been conferred by measuring TGF- bi levels The samples were derived from the rats described in Examples 1 and 2.
***Serum TGF-b1 levels:*** TGF-b₁ levels were measured by a "sandwich" ELISA using Genzyme Diagnostics kit according to manufacturers' instructions. Serum TGF-bl levels were measured in three rats from each group after each injection, on days 8 and 101.

### Measurement of TGFb₁ secreted by intestinal lymphocytes and splenocytes:

For the extraction of gut wall lymphocytes, the small intestines were removed from 2 rats each from the tolerized and the control groups on day, 101, and placed in RPMI medium supplemented with 15% FBS. The intestines were cuts into 1 cm segments, flushed with the medium, opened by cutting longitudinally and transferred into fresh medium, rinsed four times with PBS and placed in PBS (calcium and magnesium free), containing 1 mM EDTA and 1mM dithiothretol (DTT). Fragments were stirred for 30 minutes at 37°C and the exfoliated cells were harvested by decanting the PBS after tissue fragments had settled. Cells were passed through nylon wool, pelleted by centrifugation for 5 minutes, suspended in 10 ml of 40% Percoll and layered over a cushion of 70% Percoll. Cells were then centrifuged for 20 min at 600 x g and the gut wall lymphocytes at the 70%-40% interface were harvested.

For enrichment of antigen presenting cells, lymphocytes were harvested from the spleens as described above. Cells were then suspended in 4 ml of RPMI containing 5% fetal calf serum (FBS) and 4 ml of RPMI containing 5% FBS and 14.5 g% metrizamide. After centrifugation at 1800 x g for 20 min, at room temperature, the interface containing an enriched population of macrophages and dendritic cells was collected.

For determination of TGF-bl secretion, intestinal wall or splenic lymphocytes from untreated Gunn rats, rats from the control groups C and D, and rats from the tolerized group A after each injection, on days 8 and 101 (two rats from each group) were plated on tissue culture dishes (5X10⁶/10 cm plate) and grown in serum-free media. 1x10⁶ antigen presenting cells were added per plate along with 50 mg of adenoviral protein extracts as the activating antigen. After 72 hr of culturing, TGF-bl secreted into the media was quantified by ELISA as described above.

***Measurements of Serum TGFb₁ concentrations and TGFb₁ secretion in vitro:*** Serum TGFb₁ levels were increased to > 170 ng/ml after each injection of the recombinant adenovirus in rats that were given the 1 mg/day dose of adenoviral proteins before the injection of recombinant viruses (group A). In rats that received BSA or no protein before the virus injection (Groups C and D) serum TGFb₁ levels were 30-35 ng/ml (p < 0.005). The levels in normal untreated Gunn rats are 18-26 ng/ml.

*In vitro* assays for evaluation of TGFb₁ levels after exposure of splenocytes and gut wall lymphocytes to adenoviral antigens were done in two rats from each group after each virus injection. Low levels (>4 ng/ml) of TGFb₁ were present in the supernatant of splenocytes and gut wall lymphocytes cultures from the rats that were administered BSA or no protein (group C and D); similar levels of TGFb₁ were observed using gut wall lymphocytes from tolerized rats (normal levels: 2.1-3.6 ng/ml). In contrast, splenocytes from rats tolerized by feeding adenoviral proteins (group A) secreted significantly greater amounts of TGF*b*₁ (24-26 ng/ml) after exposure to viral proteins.

### Reference Example 9 Assessment of various cytokines as markers for oral tolerization

The following example demonstrates the method for establishing that tolerization has been conferred by measuring the levels of various cytokines.The samples were derived from the rats described in Examples 1 and 2.
***RT-PCR for rat cytokine mRNAs:*** These assays were performed on day 101 on two rats from each of groups A, C, D and E. After culturing the lymphocytes with the viral antigens and antigen presenting cells as described above, cells were harvested and mRNA levels for rat IL-2, 4, 6, 10, IFN-g and TGF-b₁ were determined by reverse transcription-primed polymerase chain reaction (RT-PCR One mg of RNA was used as template for each sample. Amplimers for rat glyceraidehyde-3-phosphate dehydrogenase (GPDH) were used as an internal control for the RT-PCR.
***Results of RT-PCR for rat cytokines:*** RT-PCR was performed on days 8 and 101 on RNA extracted from and gut wall lymphocyte cell cultures from the various groups. Positive bands for IL-2, 4, 10, and TGFb₁ were found in splenocytes from rats tolerized by adenoviral protein administration (group A), but not from rats that had received BSA or no protein at all (groups C or D). Gut wall lymphocytes cell cultures from the tolerized rats (group A), as well as from rats that received BSA or no proteins (groups C or D), were negative for these cytokine mRNAs. In contrast, IFNg was negative by RT-PCR in spienocytes from the tolerized rats in group A, but was found in splenocytes from rats from control groups C and D. Gut wall lymphocytes (GW) showed similar results to non-tolerized rats. IL6 was detected in all tested groups and probably behaves as a non specific acute phase reactant.

### Example 10. Oral Tolerization for Treatment of Colitis

In this example rats that had undergone oral tolerance to proteins extracted from the colons of colitis-afflicted rats demonstrated reduced pathogenesis. Tolerizing treatment was administered simultaneously with the induction of colitis.
Methods. Colitis was induced by intracolonic installation of trinitrobenzenesulfonic acid (TNBS). Treated rats received 5 oral dosages of proteins extracted from diseased colons every other day starting on the day of colitis induction. Control rats received similar doses of bovine serum albumin.
Results. Control non-tolerized rats developed severe colitis manifested by severe diarrhea end macroscopically and microscopically severe bowel disease. Feeding of colon proteins enabled induction of anti-colonimmune hyporesponsiveness manifested by: decreased diarrhea with marked decrease in the degree of colonic ulceration, intestinal and peritoneal adhesions, wall thickness and edema. There was marked reduction in the percentage of the injured colonic area out of the total colonic wall and a reduction in colon weight. Microscopic parameters were also improved in tolerized anmimals which showed a marked reduction in inflammatory response and mucosal ulceration.

The role of suppressor lymphocytes in tolerization was evaluated by testing whether lymphocytes from tolerized rats can adoptively transfer the tolerance to naive animals. Here, splenocytes were harvested from both groups of rats and transplanted into sublethally irradiated animals. The TNBS-induced colitis was markedly alleviated in recipients of splenocytes from tolerized rats. In contrast, in those animals that received lymphocytes from non-tolerized control rats, severe disease developed.

### Reference Example 11 Treatment of Graft Versus Host Disease (GVHD) through Induction of Oral Tolerance

The ability to induce tolerance in a donor towards recipient tissue can be performed through feeding of recipient lymphocytes.
Methods. In order to induce GVHD donor spleen cells will be injected intravenously into sublethally irradiated BALB/c mice. Donor mice (B10D2 males) will be fed on alternate days for 10 days with protein extracted from recipient lymphocytes. The cells will be prepared by isolating peripheral blood lymphocytes from recipient mice (Balb/c fernales). Quantitative assessment of the proteins in the cells will be made using standard methods.
**Procedures.** Ten donor mice will be fed with 5 doses of the antigens. Ten control mice will be fed with BSA. On day 12, two days after completion of the feeding, spleens will be harvested and will be transplanted in the recipient mice. The mice will be monitored for the following parameters:
   a) determination of tolerance induction by mixed lymphocyte reaction assays;
   b) induction of GVHD by skin biopsies and skin collagen determination; and
   c) induction of liver and small bowel GVHD.

## Claims

1. Colon-extracted proteins from allogeneic and autologous sources for use in the treatment of Crohn's disease.

## Patentansprüche

1. Aus dem Colon extrahierte Proteine aus allogenen und autologen Quellen zur Verwendung bei der Behandlung von Morbus Crohn.

## Revendications

1. Protéines extraites du côlon de sources allogéniques et autologues pour l'utilisation dans le traitement de la maladie de Crohn.
